# EUROPEAN PATENT SPECIFICATION

(11) **EP 2 265 305 B1**
(45) Date of publication and mention of the grant of the patent: **12.09.2012**
(21) Application number: 09725625.9
(22) Date of filing: 25.03.2009
(51) Int. Cl.: A61M 5/145

(54) **DISPENSING SYSTEM**
AUSGABESYSTEM
SYSTÈME DE DISTRIBUTION

(30) Priority: 27.03.2008 GB 0805521
(43) Date of publication of application: 29.12.2010
(73) Proprietor: The Technology Partnership PLC, Melbourn Royston Hertfordshire SG8 6EE (GB)
(72) Inventor: MCCRONE, James, Cambridgeshire CB2 9HU (GB); BURSTALL, Oliver, Cambridgeshire PE28 0PA (GB); STONE, Edwin James, Cambridgeshire (GB)
(74) Representative: Smee, Anthony James Michael
(86) International application number: PCT/GB2009/050281
(87) International publication number: WO 2009/118553

(56) References cited:
- EP-A- 1 769 814
- EP-A- 1 857 130
- WO-A-03/103758
- DE-A1- 19 627 619
- DE-C1- 10 219 750
- JP-A- 6 063 133
- US-A- 5 919 167

## Description

This invention relates to a dispensing system and, in particular, to a dispensing system which can be utilised to dispense controlled volumes of fluid from a disposable container.

It is well know to have dispensing mechanisms which use a simple reciprocating action to induce linear motion in a single direction. Such as an actuator is shown in WO03/103758, in which a shape memory alloy (SMA) actuator is used to generate a reciprocating motion by having the SMA element acting between two movable parts.

In its rest state, the spring or other biassing means applies its sufficient force to extend the SMA element, pushing the two sliding parts apart. When energised, the SMA element contracts, thereby providing sufficient force to both compress the spring and to pull the two sliding parts together. These parts (the combination of which will be described later as the "pusher") move together and translate linearly, for example, along a tube. In this way, a reciprocating motion is set up.

SMA actuators are energised by heating the SMA element above a critical heating temperature, usually achieved through resistive heating of the element by passing an electric current through it. On cooling back below the critical cooling temperature (typically lower than the heating temperature), the system returns to its rest state.

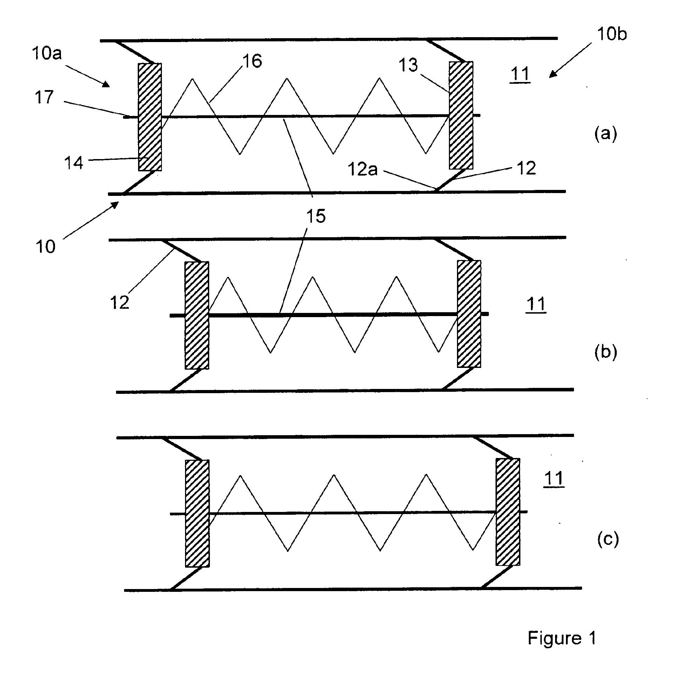

In order to convert the reciprocating motion to linear motion, the two sliding parts are made to slide preferentially in one direction. In this way, the "lead" end slides when the actuator extends and the "back" end slides when the actuator contracts, thereby resulting in a caterpillar style linear movement (see Figure 1).

Such devices however have two key limitations. Firstly, unless a facility is provided to return the pusher to the start of travel, the device can only provide a maximum travel which is equal to the length of the tube or structure that the SMA actuator is moving along. The whole assembly must then be discarded and this can be costly, given that the expensive part of the dispensing system is the SMA actuator itself. Secondly, electrical contact must be made to the pusher which is continually translating away from its start position and this can lead to difficult wiring and hence potential and more frequent failures of the electrical system.

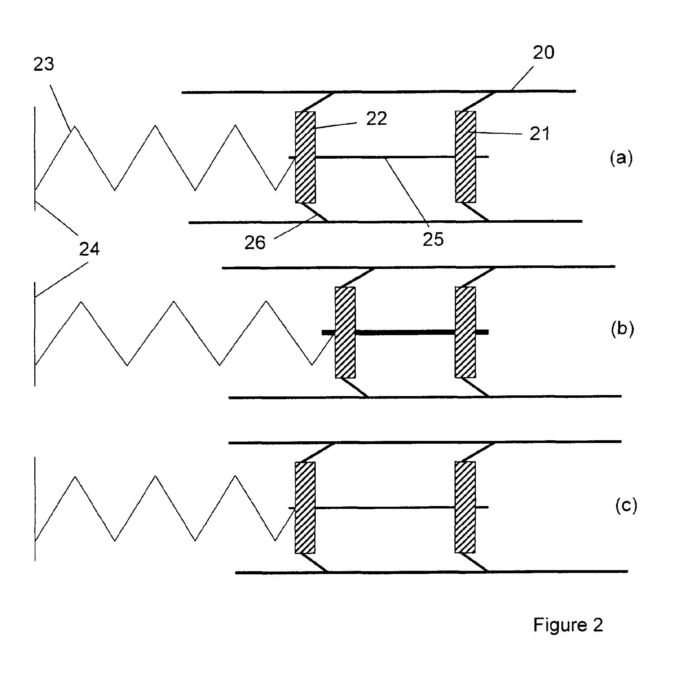

The second of these disadvantages can be addressed by an alternative configuration which can seen in, for example, US 5919167. In this example, the SMA acts between a static and a movable part, with a return spring in series with the SMA actuator. The movable part reciprocates and its motion is converted to continuous travel via a uni-directional coupling. However, such devices still require the disposal of the SMA and associated parts after all of the fluid has been dispensed as they cannot be reused (see Figure 2). Furthermore, the devices use the SMA to expel the fluid and the spring to return the SMA to its unenergised length, and this necessitates either rapid fluid expulsion (and hence large viscous loss within the device) to ensure a short (and therefore efficient) SMA heating cycle or, alternatively, a long inefficient heating cycle to minimise viscous loss.

Accordingly, the present invention aims to solve the two drawbacks identified above without introducing further disadvantages.

DE 10219750 discloses a dispensing system comprising:
a main body having an electrically driven SMA actuator;
a container for a medium to be dispensed, the container being separate from, but connectable to, the main body;
a dispenser, a portion of which is, in use, to be located within the container and movable relative thereto so as to selectively dispense a portion of the medium, the dispenser also being arranged to be connected in use to the actuator of the main body;
wherein the actuator is arranged to cause relative movement between the dispenser and the container in order to dispense a predetermined portion of the medium;
wherein the actuator comprises at least one spring.

According to the present invention such a system is characterised in that the SMA and the spring are configured such that, when the SMA is energised, the spring is compressed.

Thus, by having a main body which contains the SMA actuator connected to a dispenser, with a separate and disposable container for the medium to be dispensed, the present invention provides a solution which ensures that the disposable part is of little or negligible cost when compared to the remaining cost of the main body of the dispensing system which contains the relatively expensive SMA part.

The SMA and the spring may be configured such that medium is dispensed after the SMA is de-energised. In this way, the invention provides a configuration in which energy is stored in a spring during a rapid heating cycle and which is caused to expel the fluid at a low rate to reduce a viscous loss by the expansion of the spring.

The spring is preferably arranged to move the dispenser relative to the container after the SMA has been de-energised.

The SMA actuator may be configured to produce either an oscillating linear motion or alternatively it may produce an oscillating rotary motion. Whichever is generated, the oscillating motion is preferably converted into step-wise incremental motion of the dispenser, typically via a one-way ratchet mechanism.

In a different example, the actuator may, rather than an SMA actuator, be a piezoelectric actuator which is preferably connected to a bending beam such that, when the piezoelectric actuator is driven, the beam is caused to deflect. In this arrangement, the bending beam is preferably arranged so as to move the dispenser relative to the container after the bending beam is de-energised.

Examples of the present invention will now be described with reference to the accompanying drawings, in which :
Figure 1 illustrates the caterpillar style linear movement of the prior art;
Figure 2 illustrates the static and moveable configuration of the prior art;
Figures 3 and 4 each show a schematic representation of an embodiment of the present invention; and
Figure 5 shows a schematic representation of an example of a similar device.

In Figure 1, a dispensing tube 10 having an inlet and 10a and an outlet end 10b and holding a medium 11 therein for dispensing is provided, the tube having means, for preferentially allowing movement in one direction, in this case to the right of the drawings, whilst hindering or preventing movement in the opposite direction, i.e. to the left of the drawings. The means may include a series of sloped teeth as a ratchet mechanism, but in this embodiment, an inclined element 12 is provided on each movable element 13, 14 which achieves the same functionality. The element 12 is preferably positioned at inclined angle to the side wall and with a pre-load (e.g. by virtue of bending) so that there exits a contact force between its tip 12a and the side wall of the tube 10. Such a configuration will reduce said contact force if the movable element is moved parallel to the side wall from left to right and will increase said contact force if the movable element is moved parallel to the side wall from right to left. Hence, this results in preferential (and in the limit case, one way) movement of the movable element from left to right. Element 12 may include a series of projections around each movable element or may be formed as a continuous collar. Movable elements 13 and 14, placed at different axial locations within tube 10, are connected by means of a shape memory alloy (SMA) element 15 and a spring 16. An electrical connection 17 extends away from the shape memory alloy 15 to a power source (not shown).

Movable elements 13, 14 form a dispenser which is moveable within the tube 10 so as to dispense the fluid medium 11 from the outlet 10b of the tube. The shape memory alloy 15 and the spring 16 form the actuator which causes linear motion of the dispenser along the tube, in this case to the right in the figures.

Movement along the tube 10 is achieved with reference to Figures 1 b and 1 c. As shown in Figure 1 b, the SMA 15 is contracted (shown by a thicker line) thus causing moveable element 14 to slide to the right. Element 14 is caused to move preferentially to element 13, as the slope of element 12 permits movement preferentially to the right. When the SMA 15 is de-energised, the spring 16, which was compressed when the SMA 15 was energised, causes moveable element 13 to move to the right. Again element 13 is preferentially moved due to the shaped nature of element 12.

In this way, the dispenser is caused to move in small increments along tube 10, thereby dispensing fluid from outlet 10b.

A second known example is shown in Figures 2a to c in which a dispensing tube 20 is moveable relative to a static element 21 and a moveable element 22 of the dispenser. In this example the dispensing tube 20 is caused to move and its motion causes a plunger to be moved within a container having medium to be dispensed.

Operation of this system occurs as follows: a spring 23 is mounted between a fixed surface 24 and one side of the moveable element 22, with a shaped memory alloy element 25 connecting the moveable 22 and static 21 element. Upon energising the shaped memory alloy element 25 and hence contracting it, the moveable element 22 is drawn towards the static element 21 and, due to the shape and arrangement of the sloped element 26, the dispensing tube is also caused to move to the right in Figure 2a. This is shown in Figure 2b with the SMA actuator in the energised state and the spring 23 extended. When the SMA is de-energised, the spring returns to its at rest position, i.e. contracts, thereby pulling the moveable element 22 to the left as shown in Figure 2c. By this movement, the dispensing tube 20 is translated to the right in a stepwise motion.

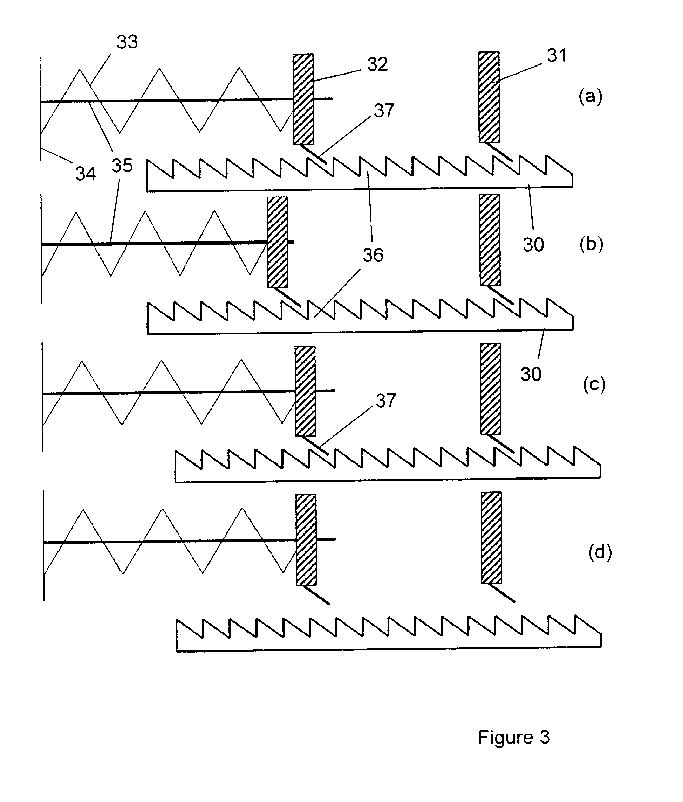

The example shown in Figures 3 to 5 show the details of solutions to the problems of the prior art.

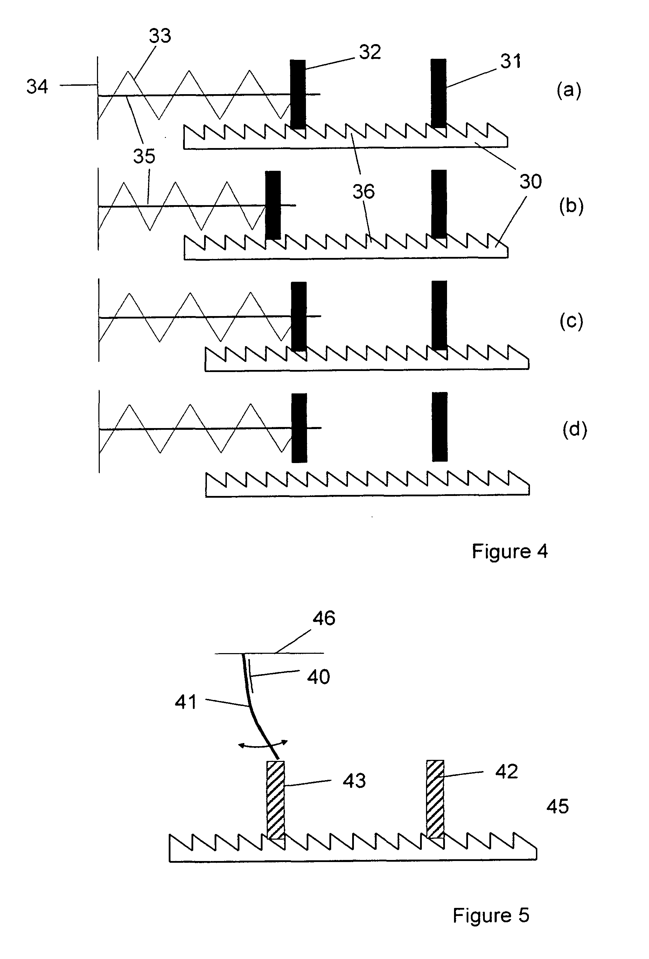

In Figures 3 and 4, a shape memory alloy plus spring actuator is provided that reciprocates, but does not translate and this leads to a simple low cost disposable components containing the medium to be dispensed which contains a means, typically in the form of a ratchet or the like, which converts the reciprocating motion into linear motion. This also is a configuration in which energy is stored in a low force spring during a rapid heating cycle and is caused to expel fluid at a low rate to reduce viscous loss when the SMA is de-energised.

This is shown in Figures 3a to d and 4a to d, in which a static element 31, to be mounted on the main body (not shown) of the dispensing system, and a moveable element 32 are arranged relative to a translating element 30 such that the translating element, which forms part of the disposable container for the medium to be dispensed can be easily removed from the static and moveable elements 31, 32, as well as from the remainder of the dispensing system.

The translating element 30 is, for example, a plunger in a static container of medium to be dispensed, such that movement of the element 30 by the SMA 35 and spring 33 combination causes liquid to be expelled from the container.

In this example, a spring 33 is connected between a static element 34 and one side of the moveable element 32, as was the situation in Figure 2a. However, in this example, the SMA 35 is also connected between a static surface 34 and the moveable element 32. The translating element 30 is provided, in Figure 3, with a sloped element 37 similar to that described with reference to Figures 1 and 2, and in Figure 4, with a series of teeth 36. Each mechanism preferentially allow movement to the left in the figures, whilst preferentially preventing movement to the right.

Thus, in operation, the SMA is energised and contracts, thereby drawing the moveable element 32 to the left in the drawings. This is the rapid heating cycle and is an efficient way of storing energy in the low force spring 33. When the SMA is de-energised, the spring starts to extend back to its at rest position, thereby forcing moveable element 32 to the right and hence moving the translating element 30 also to the right. This expansion of the spring is done at a slower rate than the compression of the spring, and thereby ensures that this stage produces less viscous loss than known configurations, as the fluid is dispensed. In order to be able to reuse the dispenser which is formed by the SMA 35, the spring 33, the static part 31 and the moveable part 32, a user simply has to disengage the translating tube from the static 31 and moveable 32 element as shown in Figures 3d and 4d.

Thus, in this embodiment, the SMA and spring actuator is reusable, since both the SMA element and the spring are capable of withstanding thousands of use cycles. This is highly beneficial from a cost and a waste point of view. Furthermore, the electrical contacts to the SMA do not undergo a continuous translation, but rather only oscillate between two positions, thereby providing easier design and more reliable connection.

The low cost of the disposable part enables the invention to be used in cost sensitive application areas such as consumer products. The compact nature of the invention allows it to be packaged within other systems, e.g. within the handle of a toothbrush.

The disposable parts could, for example, include a fluid reservoir for the dispensing of liquids, gels, creams, powders or tablets and may include the dispensing of, for example, food stuffs in liquid, powder or tablet form, personal care products such as cosmetics e.g. lipsticks or creams, hand cleaners and sanatisers or toothpaste or even shaving products, pet care such as pet food on a timer, cleaning products, air freshners, medicinal tablets or stationary. The reciprocating motion can be converted into quasi-continuous linear motion using a variety of methods. These include the toothed ratchet and pawls as shown in Figure 3, in which one pawl is held static whilst the other is oscillated back and forth. Alternatively, this could be achieved using friction and a pawl, whereby the static pawl in the examples of Figures 3 or 4 could be replaced by a simple frictional engagement. A third alternative is that the reciprocating motion could be used to drive the rotation of, for example, a threaded rod causing a nut on the rod which is constrained from rotating to translate along its length. This can equally be configured such that the nut is rotated and the rod, which is constrained from rotation, translates. In any event, a reciprocating motion is translated into quasi-continuous linear motion.

All of the above systems can be made through simple low cost manufacturing methods such as injection molded plastic. The reciprocation could alternatively be rectified directly by using a diaphragm type pump and this part can still be configured to be separable from the main actuator.

In a different example shown in Figure 5, a piezoelectric actuator 40 is connected to a bending beam 41 mounted on the main body 46, the beam being activated to cause movement of the beam in one direction and deactivated to cause the beam to return to its at rest position. This can be configured to provide the necessary reciprocating motion shown by arrow 42 in order to drive the oscillating part 43. A static part 44, in conjunction with a dispensing tube 45 is also provided and this mechanism operates much in the same way as that of Figures 3 and 4 and further description of that operation is unnecessary.

## Claims

1. A dispensing system comprising:
a main body having an electrically driven SMA actuator (31,32,33,35);
a container (10,20) for a medium to be dispensed, the container being separate from, but connectable to, the main body;
a dispenser (30), a portion of which is, in use, to be located within the container and moveable relative thereto so as to selectively dispense a portion of the medium, the dispenser also being arranged to be connected in use to the actuator (31,32,33,35) of the main body;
wherein the actuator is arranged to cause relative movement between the dispenser and the container in order to dispense a predetermined portion of the medium, wherein the actuator comprises at least one spring; **characterised in that**
the SMA (35) and the spring (33) are configured such that, when the SMA is energised, the spring is compressed.

2. A system according to claim 1, wherein the SMA (35) and the spring (33) are configured such that medium is dispensed after the SMA is de-energised.

3. A system according to claim 2, wherein the spring (33) is arranged so as to move the dispenser (30) relative to the container after the SMA (35) is de-energised.

4. A system according to any one of the preceding claims, wherein the actuator of the main body produces oscillating linear motion.

5. A system according to any one of claim 1 to 4, wherein the actuator (31,32,33,35) of the main body produces oscillating rotary motion.

6. A system according to claim 5, wherein the oscillating rotary motion is converted into oscillating linear motion.

7. A system according to any of claims 4 to 6, wherein the oscillating motion is converted into step-wise incremental motion of the dispenser (30).

8. A system according to any one of the preceding claims, wherein the actuator (31,32,33,35) and the dispenser (30) are connected via a one way ratchet mechanism.

## Patentansprüche

1. Ausgabesystem, das Folgendes umfasst:
einen Hauptkörper mit einer elektrisch angetriebenen FGL-Betätigungsvorrichtung (31, 32, 33, 35);
einen Behälter (10, 20) für ein auszugebendes Medium, wobei der Behälter von dem Hauptkörper getrennt aber damit verbindbar ist;
eine Ausgabevorrichtung (30), von der ein Anteil dazu vorgesehen ist, sich in Gebrauch in dem Behälter zu befinden und relativ dazu bewegbar zu sein, um selektiv einen Anteil des Mediums auszugeben, wobei die Ausgabevorrichtung außerdem dazu angeordnet ist, in Gebrauch mit der Betätigungsvorrichtung (31 ,32, 33, 35) des Hauptkörpers verbunden zu sein;
wobei die Betätigungsvorrichtung dazu angeordnet ist, Relativbewegung zwischen der Ausgabevorrichtung und dem Behälter zu bewirken, um einen vorherbestimmten Anteil des Mediums auszugeben, wobei die Betätigungsvorrichtung mindestens eine Feder umfasst; **dadurch gekennzeichnet, dass**
die FLG (35) und die Feder (33) derart ausgebildet sind, dass, wenn die FLG unter Spannung steht, die Feder zusammengedrückt ist.

2. System nach Anspruch 1, wobei die FLG (35) und die Feder (33) derart ausgebildet sind, dass das Medium ausgegeben wird, nachdem die FLG spannungsfrei geschaltet wird.

3. System nach Anspruch 2, wobei die Feder (33) dazu angeordnet ist, die Ausgabevorrichtung (30) relativ zu dem Behälter zu bewegen, nachdem die FLG (35) spannungsfrei geschaltet wird.

4. System nach einem der vorangehenden Ansprüche, wobei die Betätigungsvorrichtung des Hauptkörpers lineare Schwingbewegung erzeugt.

5. System nach einem der Ansprüche 1 bis 4, wobei die Betätigungsvorrichtung (31, 32, 33, 35) des Hauptkörpers Kreisschwingbewegung erzeugt.

6. System nach Anspruch 5, wobei die Kreisschwingbewegung in lineare Schwingbewegung umgewandelt wird.

7. System nach einem der Ansprüche 4 bis 6, wobei die Schwingbewegung in schrittweise inkrementelle Bewegung der Ausgabevorrichtung (30) umgewandelt wird.

8. System nach einem der vorangehenden Ansprüche, wobei die Betätigungsvorrichtung (31, 32, 33, 35) und die Ausgabevorrichtung (30) über einen Einweg-Ratschenmechanismus verbunden sind.

## Revendications

1. Système de distribution comprenant :
un corps principal comportant un actionneur en AMF entraîné électriquement (31, 32, 33, 35) ;
un contenant (10, 20) pour un milieu à distribuer, le contenant étant séparé du corps principal mais pouvant y être raccordé ;
un distributeur (30), dont une portion, en utilisation, doit être située dans le contenant et mobile par rapport à celui-ci de façon à distribuer sélectivement une portion du milieu, le distributeur étant également disposé pour être raccordé en utilisation à l'actionneur (31, 32, 33, 35) du corps principal ;
l'actionneur étant disposé pour provoquer un mouvement relatif entre le distributeur et le contenant de façon à distribuer une portion prédéterminée du milieu, l'actionneur comprenant au moins un ressort ; **caractérisé en ce que**
l'AMF (35) et le ressort (33) sont configurés de sorte que, lorsque l'AMF est alimenté en énergie, le ressort est comprimé.

2. Système selon la revendication 1, dans lequel l'AMF (35) et le ressort (33) sont configurés de sorte que le milieu est distribué après la mise hors tension de l'AMF.

3. Système selon la revendication 2, dans lequel le ressort (33) est disposé de façon à déplacer le distributeur (30) par rapport au contenant après la mise hors tension de l'AMF (35).

4. Système selon l'une quelconque des revendications précédentes, dans lequel l'actionneur du corps principal produit un mouvement linéaire d'oscillation.

5. Système selon l'une quelconque des revendications 1 à 4, dans lequel l'actionneur (31, 32, 33, 35) du corps principal produit un mouvement rotatif d'oscillation.

6. Système selon la revendication 5, dans lequel le mouvement rotatif d'oscillation est converti en mouvement linéaire d'oscillation.

7. Système selon l'une quelconque des revendications 4 à 6, dans lequel le mouvement d'oscillation est converti en mouvement incrémentiel par palier du distributeur (30).

8. Système selon l'une quelconque des revendications précédentes, dans lequel l'actionneur (31, 32, 33, 35) et le distributeur (30) sont raccordés via un mécanisme à cliquet antiretour.
